**Europäisches Patentamt**

**European Patent Office**

**Office européen des brevets**

(11) Veröffentlichungsnummer: **0 123 142**
**B1**

(12) **EUROPÄISCHE PATENTSCHRIFT**

(45) Veröffentlichungstag der Patentschrift:
**09.12.87**

(21) Anmeldenummer: **84103164.4**

(22) Anmeldetag: **22.03.84**

(51) Int. Cl.⁴: **A 61 K 31/40**

(54) **Verfahren zur Stabilisierung von Hämatin.**

(30) Priorität: **28.03.83 DE 3311288**

(43) Veröffentlichungstag der Anmeldung:
**31.10.84 Patentblatt 84/44**

(45) Bekanntmachung des Hinweises auf die Patenterteilung:
**09.12.87 Patentblatt 87/50**

(84) Benannte Vertragsstaaten:
**AT BE CH DE FR GB IT LI LU NL SE**

(56) Entgegenhaltungen:
**Chemical Abstracts Band 55, Nr. 19, 18. September
1961, Columbus, Ohio, USA; H.C. GOMES OLIVEIRA et
al. "Action of x-rays on hemin", Spalten 19008l-19009a
Chemical Abstracts Band 97, Nr. 1, 5. Juli 1982,
Columbus, Ohio, USA; D.V. GODIN et al. "Studies on the
interaction of barbiturates with reactive oxygen
radicals: Implications regarding barbiturate protection
against cerebral ischemia", Seite 80, Spalten 1, 2,
Abstract Nr. 695h**

(73) Patentinhaber: **BEHRINGWERKE Aktiengesellschaft,
Postfach 1140, D-3550 Marburg 1 (DE)**

(72) Erfinder: **Zilg, Harald, Dr., Am Kornacker 31,
D-3550 Marburg (DE)**

(74) Vertreter: **Meyer-Dulheuer, Karl-Hermann, Dr. et al,
HOECHST Aktiengesellschaft Zentrale Patentabteilung
Postfach 80 03 20, D-6230 Frankfurt/Main 80 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein Verfahren zur Stabilisierung von Hämatin in fester oder gelöster Form. Das stabilisierte Hämatin kann als Arzneimittel angewendet werden, vorzugsweise in der Behandlung bestimmter Formen von Porphyrie.

Nach dem Stand der Technik werden für die Anwendung am Patienten geeignete Hämatin-Infusionslösungen unmittelbar vor dem Zeitpunkt des Bedarfs aus dem Wirkstoff Hämatin unter Einhaltung bestimmter Vorsichtsmassnahmen, insbesondere hinsichtlich Pyrogenfreiheit, hergestellt, ein Verfahren, das nicht in jeder Klinik zu verwirklichen ist (C.A. Pierach, Der Internist 22 (1981) 726–732). Darüber hinaus ist auch eine Technik bekannt, eine Hämatinpräparation in lyophilisierter Form herzustellen unter Zusatz von Mannit; auf diesem Wege kann durch Rekonstitution mit einem geeigneten Lösungsmittel eine Hämatinlösung mit einer gewissen, jedoch unbefriedigenden Haltbarkeit hergestellt werden (J.M. Lamon et al., Medicine 58 (3) (1979) 252–269). Aus Arch. Int. Pharmacodyn 1961, CXXXI, Nr. 1–2, Seiten 236 bis 238 ist bekannt, dass Albumin eine schützende Wirkung auf Hämin gegen den Einfluss von Röntgenstrahlen ausübt.

Überraschenderweise wurde nunmehr gefunden, dass Albumin Hämatinpräparationen sowohl in Lösung als auch in lyophilisierter Form zu stabilisieren vermag.

Gegenstand der Erfindung ist daher ein Verfahren zur Stabilisierung einer Hämatinpräparation, dadurch gekennzeichnet, dass dem Hämatin Albumin zugesetzt wird.

Zu diesem Zweck kann eine Hämatinlösung mit einer Albuminlösung gemischt und die Mischung zur Trockene gebracht werden. Es kann aber auch Hämatin und Albumin jeweils in fester Form zusammengebracht und gegebenenfalls vermischt werden. Es kann auch die eine Komponente in fester Form mit einer Lösung der anderen Komponente zusammen- und zur Trockene gebracht werden.

Hämatin als Wirkstoffkomponente einer derartigen Präparation wird nach an sich bekannten Verfahren hergestellt (H. Fischer, Org. Synthesis Collective Volume, J. Wiley ed., New York (1955), 3, 442), wobei Ausgangsmaterialien tierischen, vorzugsweise aber menschlichen Ursprungs, hier wiederum vorzugsweise «verfallene» Blutkonserven verwendet werden.

Als stabilisierende Komponente gelangt Serumalbumin humanen Ursprungs zum Einsatz, wobei vorzugsweise Albumin verwendet wird, das nach Europäischer oder anderen Pharmakopöen eine Erhitzung auf 60°C in Gegenwart von geeigneten Stabilisatoren erfahren hat. Darüber hinaus können aber auch andere Serumalbuminpräparationen humanen Ursprungs zum Einsatz kommen.

Das Mengenverhältnis von Hämatin und Albumin wird so gewählt, dass nach Rekonstitution der getrockneten, vorzugsweise lyophilisierten Mischung mit einem Lösungsmittel, besonders Wasser, das zur Einstellung der Isotonie Salze enthalten kann, die Hämatinkonzentration 0,5 bis 10 g/l, vorzugsweise aber 2 bis 7 g/l, und die Albuminkonzentration 2 bis 200 g/l, vorzugsweise aber 7 bis 50 g/l, beträgt. Hierbei kann so verfahren werden, dass Hämatin in Gegenwart einer geeigneten Puffersubstanz, vorzugsweise aber in Abwesenheit einer Puffersubstanz, im alkalischen Milieu, vorzugsweise im pH-Bereich von 9–12, unter Zugabe einer Natriumhydroxidlösung gelöst wird. Nach Einstellen eines pH-Wertes zwischen 7 und 9, vorzugsweise zwischen 7,5 und 8,5, kann dann eine Humanalbuminlösung zugeben werden. Danach kann die Salzkonzentration eingestellt werden. Es wird vorzugsweise eine physiologische NaCl-Konzentration im rekonstituierten Endprodukt durch Verwendung eines geeigneten Lösungsmittels angestrebt. Die Mischung von Hämatin und Albumin wird zur Trockene gebracht, vorzugsweise nach Abfüllen des gewünschten Volumens in einen Endbehälter und lyophilisiert.

Trocken vorliegendes Hämatin und Albumin können aber auch in fester Form vor dem Abfüllen oder im Endbehälter zusammengebracht und gegebenenfalls gemischt werden.

Der Vorteil der Verwendung von Albumin gegenüber anderen Stabilisatoren geht aus dem Verlauf des Abfalls des Hämatingehaltes mit der Zeit im Anschluss an die Rekonstitution des Lyophilisates hervor, wie in der folgenden Tabelle zusammengestellt. Hierbei wurde Hämatin als Bispyridin-Ferroprotoporphyrin IX-Komplex bei einer Wellenlänge von 554 nm photometrisch bestimmt (K.G. Paul et al., Acta chem. scand. (1953) 7, 1284–1287). Die Lösungen enthielten jeweils 4 g/l (= 100%) Hämatin zum Zeitpunkt der Herstellung sowie die angegebenen Mengen Stabilisator.

Tabelle: Hämatingehalt nach Lagerung bei 4°C
(Ausgangswert bei Rekonstitution = 100%)

| Stabilisator | Zeit nach Rekonstitution | | | |
| --- | --- | --- | --- | --- |
| | 6 h | 24 h | 72 h | 90 Tage |
| Albumin 25 g/l | 99,0 | 98,7 | 97,6 | 96,2 |
| Glycin 10 g/l | 89,2 | 81,5 | 77,4 | 79,9 |
| Mannit 4 g/l | 96,0 | 83,5 | 79,5 | 79,2 |
| Mannit 10 g/l | 97,6 | 86,9 | 79,8 | 79,5 |

Wie aus der Tabelle hervorgeht, verhindert Albumin insbesondere den bei anderen Stabilisatoren in der Anfangsphase zu beobachtenden Abfall im Hämatingehalt, wobei die Gegenwart der Stabilisatoren die photometrische Bestimmung nicht beeinflusst. Darüber hinaus verändert sich in Gegenwart von Albumin auch die Lage des Absorptionsmaximums in diesem Zeitraum nicht, während Hämatinlösungen ohne Stabilisatorzusatz sowie mit einem Zusatz an Glycin oder Mannit eine hypsochrome Verschiebung von 2–3 nm bereits nach 24 h erfahren.

Insgesamt stellt die Stabilisierung mit Albumin einen Fortschritt für die praktische Handhabung einer Hämatinpräparation in der Klinik dar, da für Rekonstitution und Anwendung am Patienten ein längerer Zeitraum zur Verfügung steht, in dem die Wirkstoffkonzentration erhalten bleibt. Beim Vergleich mit einer unstabilisierten Hämatinlösung erwies sich albuminstabilisiertes Hämatin in Prüfungen auf lokale Verträglichkeit (intravenöse Injektion in die gestaute Ohrrandvene des Kaninchens) als deutlich verträglicher.

Die therapeutische Anwendung von Hämatin in Fällen von bestimmten Porphyrien ist ein bekanntes Verfahren. Hämatin wirkt hierbei im Sinne einer nagtiven Feed-back-Kontrolle regulierend auf die Synthese des ersten Enzyms der Tetrapyrrolbiosynthese, nämlich der δ-Aminolävulinsäuresynthetase ein. Genetisch bedingt können Mangelzustände an bestimmten Enzymen der Tetrapyrrolbiosynthese bestehen, die im latenten Zustand zu keinen Krankheitssymptomen führen. Eine Porphyrie kann in solchen Patienten durch verschiedene Faktoren wie Intoxikationen, Stress, Hungerzustände u.a. induziert werden und sich durch Anhäufung toxischer Porphyrinzwischenstufen zu einer lebensbedrohenden Erkrankung ausweiten. Die schnelle Verfügbarkeit einer Hämatinpräparation ist bei diesen relativ seltenen Erkrankungen auch ein logistisches Problem für die behandelnde Klinik. Hier wird mit einem stabilen lyophilisierten Präparat, das aus dem Notfalldepot entnommen werden kann und das nach Rekonstitution bei der Anwendung keinen einschränkenden Stabilitätsbetrachtungen zu unterliegen braucht, eine Lücke geschlossen:

Die Erfindung wird an nachstehendem Beispiel erläutert.

Beispiel

3 g Hämatin werden in 200 ml 0,05 N NaOH bei Raumtemperatur suspendiert. Während des Lösevorganges wird der pH durch weitere Zugabe von NaOH auf pH 10,5 gehalten. Nach Ablauf von einer Stunde wird vom Ungelösten abzentrifugiert und filtriert. Die Hämatinkonzentration wird auf 12,5 g/l eingestellt durch Zugabe von Wasser, und der pH durch 0,5 N HCl auf 9,5 eingestellt. Zu 100 Volumenanteilen Hämatinlösung werden 37,5 Volumenanteile einer Albuminlösung von 200 g/l zugegeben, wobei im Falle eines Absinkens des pH's unter 8,0 entsprechend NaOH-Lösung zugegeben wird. Durch Zugabe gesättigter NaCl-Lösung wird eine Osmolalität von 135 mOsm/kg eingestellt. Nach Sterilfiltration werden Infusionsflaschen mit 36 ml der erhaltenen Lösung gefüllt und lyophilisiert. Als Rekonstitutionslösung sind 75 ml einer Lösung von 6 g/l NaCl vorzusehen. Im rekonstituierten Produkt liegt eine Hämatinkonzentration von 4 g/l und eine Albuminkonzentration von 25 g/l vor.

**Patentansprüche**

1. Verfahren zur Stabilisierung einer Hämatinpräparation, dadurch gekennzeichnet, dass dem Hämatin Albumin zugesetzt wird.

2. Verfahren nach Anspruch 1, dadurch gekennzeichnet, dass das Gewichtsverhältnis von Hämatin und Albumin im Bereich von 1:400 bis 5:1, vorzugsweise 1:25 bis 1:1, liegt.

3. Mittel, enthaltend Hämatin und Albumin im Gewichtsverhältnis von 1:400 bis 5:1, vorzugsweise 1:25 bis 1:1.

4. Mittel nach Anspruch 3 in trockener Form.

**Claims**

1. A process for the stabilization of a hematin product, which comprises adding albumin to the hematin.

2. The process as claimed in claim 1, wherein the weight ratio of hematin to albumin is in the range from 1:400 to 5:1, preferably 1:25 to 1:1.

3. An agent containing hematin and albumin in a weight ratio of 1:400 to 5:1, preferably 1:25 to 1:1.

4. An agent as claimed in claim 3, in dry form.

**Revendications**

1. Procédé de stabilisation d'une préparation d'hématine caractérisé en ce que l'on ajoute de l'albumine à l'hématine.

2. Procédé selon la revendication 1, caractérisé en ce que le rapport pondéral de l'hématine à l'albumine est compris entre 1:400 et 5:1, de préférence entre 1:25 et 1:1.

3. Médicament contenant de l'hématine et de l'albumine dans un rapport pondéral de 1:400 à 5:1, de préférence de 1:25 à 1:1.

4. Médicament selon la revendication 3 sous forme sèche.